# EUROPEAN PATENT APPLICATION

(11) **EP 3 375 483 A1**
(43) Date of publication of application: **19.09.2018**
(21) Application number: 16863715.5
(22) Date of filing: 28.10.2016
(51) Int. Cl.: A61N 1/375

(54) **CONNECTOR BLOCK FOR IMPLANTABLE ELECTRICAL-STIMULATION DEVICES AND SPECIFIC TOOL FOR USE IN PROCEDURES FOR REPLACING SAID DEVICES HAVING THE CONNECTOR BLOCK**

(30) Priority: 10.11.2015 ES 201531622; 18.01.2016 ES 201630047
(71) Applicant: Fernández Quesada, Fidel, 18008 Granada (ES); Palomino Guzmán, Miguel, 18008 Granada (ES)
(72) Inventor: Fernández Quesada, Fidel, 18008 Granada (ES); Palomino Guzmán, Miguel, 18008 Granada (ES)
(74) Representative: Munoz Garcia, Antonio
(86) International application number: PCT/ES2016/070768
(87) International publication number: WO 2017/081341

(57) **Abstract**

The invention relates to a connector block for implantable electrical stimulation devices, and to a specific tool for use in procedures for replacing said devices having the connector block. The connector block (1) is formed by a body (2) coupled to an impulse generator (3) housing connections (5) for electrodes (6) and securing screws (7) protected in a valve (8), and comprises at least one auxiliary port (9), with an auxiliary valve (8'), for inserting a stylus (11) or a dynamometric key (10) for pressing the connection (5). The tool (100) comprises an insulating main body (101) having a gripping part, with a hollow, cylindrical front part (102) for pushing that has an exterior width (D) for inserting into the auxiliary port (9), and a longitudinal duct (103) for inserting the stylus (11) therethrough.

## Description

As expressed by the title of the present specification, the invention relates to a connector block for implantable electrical stimulation devices and to a specific tool for use in procedures for replacing said devices having said connector block providing the intended function thereof with advantages and features that will be described in detail below and entail a significant improvement over the current state of the art.

More specifically, the object of the invention resides on one hand in the connector block which is incorporated in the impulse generator of an implantable electrical stimulation device, preferably a cardiac device, although it can also be of a neuron-type device or other devices, such as pacemakers, defibrillators, resynchronizers, neurostimulators, or others, and which houses connections and set screws for the electrodes and the battery of said device, having the particularity of incorporating an auxiliary port in the connector block which advantageously provides safety and ease of disconnection in prosthesis replacement operations, a second aspect of the present invention being a specific tool for use in procedures for replacing said implantable electrical stimulation devices provided with the connector block having an auxiliary port, which is specifically designed for being coupled to a stylet and making the disconnection of the electrodes in such procedures easier and safe, allowing the two main attributes of said device having an "auxiliary port" to be carried out simultaneously: serving as a subsequent thrusting path with respect to the electrode with adherence or a certain degree of locking and assuring patient stimulation throughout the entire procedure.

### FIELD OF APPLICATION OF THE INVENTION

The field of application of the present invention is encompassed within the sector of the industry dedicated to the manufacture of medical apparatus and devices, focusing particularly on the field of electrical stimulation devices and systems designed as implantable prostheses.

### BACKGROUND OF THE INVENTION

As is known, a cardiac stimulation device, a pacemaker, a defibrillator is an implantable prosthesis capable of detecting the electromechanical activity of the heart and sending, through electrodes, sufficient energy to cause cardiac contraction and thereby prevent asystole if no electromechanical activity is detected.

To that end, an electrical stimulation system comprises the following elements:
- An impulse generator containing the battery and memory and control circuits, connector block, etc.
- One or more electrodes formed by metal filaments and insulation systems. They are the physical support through which energy and information travel between the impulse generator and the structure to be stimulated.

Modern impulse generators have a volume of about 10 cubic centimeters and incorporate different automatisms, some for safety reasons, others for obtaining the best clinical benefit from the particular conditions of the patient, or another for simply optimizing device consumption and prolonging its service life.

In turn, the connector block houses the connections and set screws for the electrodes and the battery. They are usually manufactured in polyurethane or silicone. The connector block has been gradually adapted to the technological evolution of electrodes and connections. There is in the current systems a standardized IS1 connection which assures compatibility between different manufacturers.

For safety purposes, the set screws are tightened with a dynamometric key and furthermore protected by a valve preventing the passage of fluids into the connection.

As indicated, the electrode is the physical support putting the cardiac tissue in contact with the impulse generator which performs two functions, i.e., stimulation, if necessary, and detection of contraction-linked electrical activity.

The electrodes can be monopolar or bipolar and use different alloys in their leads; the most widely used insulators are silicone and polyurethane.

However, all these electrodes have something in common, i.e., they have inside the inner lead a lumen designed so as to be able to direct the electrode to the desired position with the help of a metal guide or stylet. The stylet is in physical and electrical contact with the inner lead.

On the other hand, it must be pointed out that the battery of the pacemaker has a service life of about ten years. After this time, the impulse generator is replaced, keeping the previously implanted electrode.

This surgical procedure consists of opening the fibrotic sac where the device is housed and debriding the body from the electrode, releasing it from adherence. Next, the clamping screws are loosened, the pacemaker to be replaced is disconnected, and the new device is connected.

The procedure is conceptually and basically very simple; however, they do usually present two common types of problems:
- Problems relating to the release of the electrode. The surgeon must be careful when releasing perielectrode fibrosis to prevent damaging the insulators and leads. Even so, while loosening the clamping screws and trying to pull in order to disconnect the electrode, difficulties tend to arise due to locked screws, the presence of fluids inside the connector block, adherence caused by interaction between the silicones of the cable and the inner portion of the connector block, and the formation of vacuum in the distal portion of the connector block. Under these circumstances, if it is withdrawn too forcefully, there is a risk of the insulator wearing away or the leads being affected.
- Problems relating to the clinical situation of the patient. Many patients whose device has to be changed have no escape beat. These patients are called "pacemaker-dependent" patients meaning that their heart contracts depending entirely on the stimulation provided by the pacemaker. In the absence of pacemaker upon disconnecting it, the patient will experience transient asystole, and, as a likely consequence, the onset of "Stokes-Adams syndrome", ventricular arrhythmias, etc.

Transient asystole while changing the impulse generator is a risk situation that must be avoided. Medications enhancing the emergence of intrinsic activity are commonly used in an operating room, and for the most extreme cases, heart beat is assured during the procedure with the installation of an external pacemaker.

It would therefore be desirable to be able to have a cardiac stimulation device having an improved connector block to prevent both types of problems, the objective of the present invention being the development of said connector block, as well as a specific tool, which allows achieving the desired objectives simultaneously: help in the mechanical unlocking of the electrode and safety given that stimulation can be assured throughout the entire procedure.

On the other hand and as a reference to the current state of the art, it must be indicated that the applicant at least is unaware of the existence of any other connector block assembly for cardiac stimulation devices and tool or similar invention having technical, structural and constitutive features similar to the features of the invention herein proposed and claimed.

### DESCRIPTION OF THE INVENTION

The connector block for implantable electrical stimulation devices and specific tool for use in procedures for replacing said devices having said connector block proposed by the invention are therefore configured as a remarkable novelty within its field of application, since the objectives indicated above as ideal are specifically and satisfactorily achieved according to its implementation, with the characterizing details which make it possible and distinguish it being conveniently included in the final claims accompanying the present description.

Specifically, as discussed above the invention proposes a connector block of the type which is intended for being incorporated in the casing of the impulse generator of an electrical stimulation device and houses the connections and set screws for the electrodes and the battery of said device, having as an essential feature the particularity of having an auxiliary connection port which basically provides two advantageous benefits: greater ease of disconnection in prosthesis replacement operations or in those situations requiring same as it serves as a thrusting path with respect to the electrode; and safety in prosthesis replacement operations on dependent patients.

To that end, said auxiliary port of the connector block of the invention is formed as an opening in the rear portion of the connector, i.e., the portion opposite the inlet/outlet portion of the electrode, and is provided with a valve having similar dimensions and characteristics as the valve of the set screws which it incorporates to prevent fluid from entering the connection.

The existence of this port allows, on one hand, destroying the vacuum in the cavity and provides the opportunity to thrust the end of the electrode from the back to disconnect same. To that end, the same dynamometric key which is supplied with each device for tightening said screws can be used. This additional thrust on the end of the electrode provides sufficient force to overcome adherence and achieve unlocking.

Furthermore, in problems relating to asystole prevention, the pulse of the patient can be assured throughout the procedure using the auxiliary port. To that end, 20 to 25 cm of a stylet without a gripping part are inserted through the inlet of said port into the lumen of the electrode. The stylet is in electrical contact with the inner lead (cathode).

Measurement cables are connected, the cathode being connected to the stylet and the anode to the skin. Stimulation is activated and it is assured that the analyzer is programmed at frequency higher than the pacemaker operating frequency. Monopolar stimulation at the frequency programmed in the analyzer is obtained. Stimulation from the stylet is thereby assured.

Once stimulation is assured, the electrode is removed, leaving a part of the stylet accessible through the front portion of the connector block.

The position for cathode stimulation through the stylet is changed from the rear portion of the connector to the portion before same. The operation is simple, quick, and devoid of uncertainty.

With the stimulation assured, the stylet is unthreaded from the connector of the pacemaker to be replaced and threaded in the connector of the new pacemaker.

Stimulation is assured by putting the cathode back in the rear portion of the stylet, i.e., after the connector of the new generator and the connection of the electrode to the new generator is made.

The use of this feature allows the procedure to be performed in a quick, simple, and risk-free manner.

However, to make said operations easier, the invention contemplates the use of a specific tool for being coupled to the stylet and for making disconnection of the electrodes easier and safe, simultaneously allowing it to serve as a subsequent thrusting path with respect to the electrode and to assure patient stimulation throughout the entire procedure.

To that end, said tool consists of a body made of plastic formed by way of a gripping part having dimensions similar to a dynamometric key, in which body there has been cut a longitudinal conduit allowing the passage of the semi-rigid stylet therethrough, as well as the sliding thereof.

Said body in turn has a front portion referred to as a thruster consisting of a thin cylinder made of the same material, said thruster is also hollow so that the stylet can also go through same and has an outer diameter suitable to allow the passage thereof through the auxiliary port of the connector block of the device to enable applying thrust on the distal end of the implanted electrode.

A way to maintain stimulation throughout the entire procedure is thereby assured by inserting the stylet into the tool and then through the auxiliary port into the implanted electrode.

By sliding the tool on the stylet and also through the auxiliary port, thrust to unlock the electrode is achieved without the stimulation ever being compromised.

If the patient is pacemaker-dependent, heart stimulation can be achieved by means of connection to the stylet and conduction through the inside of the electrode, and the tool which is movable along the stylet thrusts with the front thrusting portion the locked cable, so this contingency is solved in the same action and with a simple operation that is devoid of uncertainty.

The described connector block for implantable electrical stimulation devices and specific tool for use in procedures for replacing said devices having said connector block therefore represent an innovation having structural and constitutive features that were unknown up until now, and these reasons, combined with its practical usefulness, give the invention sufficient grounds for obtaining the exclusive right that is sought.

### DESCRIPTION OF THE DRAWINGS

To complement the description that is being made and for the purpose of aiding to better understand the features of the invention, a set of drawings is attached to the present specification as an integral part thereof in which the following has been depicted with an illustrative and non-limiting character:
Figure 1 shows a schematic view of an example of an electrical stimulator device for which the connector block object of the invention is intended, where the main parts that it comprises can be seen.
Figure 2 shows a schematic view of an example of a conventional connector block, where the configuration and elements comprised in the connector blocks known in the state of the art can be seen.
Figure 3 shows another schematic view of an example of the connector block for electrical stimulation devices according to the invention, where the parts and elements which it comprises and which distinguish it can be seen, said block being depicted with the electrode being included.
Figures 4 and 5 show respective perspective views of the way of using the connector block for electrical stimulation devices object of the invention for extracting, in Figure 4, the electrode from the connection by thrusting with a conventional dynamometric key, and for replacing, in Figure 5, the impulse generator in patients with a risk of asystole.
Figure 6 shows a schematic perspective view of an example of the specific tool for use in procedures for replacing devices having the connector block object of the invention, depicted once it has been incorporated to the stylet, where the main parts that it comprises can be seen.
Figure 7 shows a perspective view of the way of using the tool for disconnecting electrodes in a pacemaker provided with the connector block object of the invention.

### PREFERRED EMBODIMENT OF THE INVENTION

In view of the mentioned drawings and according to the numbering used, an example of the connector block for implantable electrical stimulation devices and of the specific tool for procedures for replacing said devices having a connector block, which comprise the parts and elements indicated and described in detail below, can be seen therein.

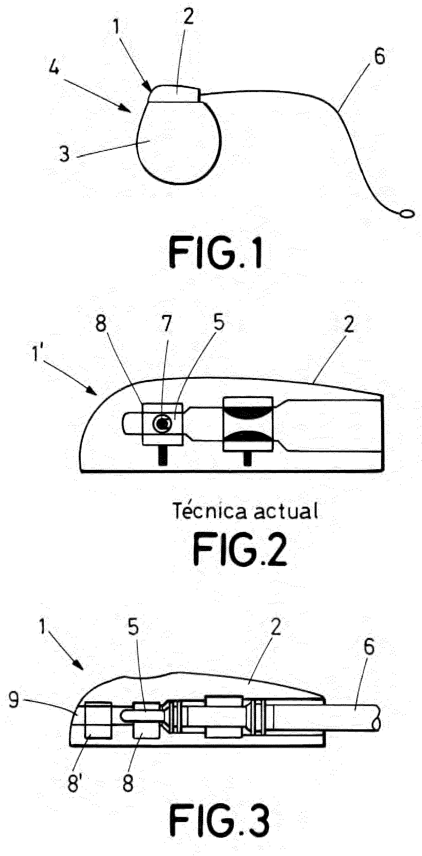

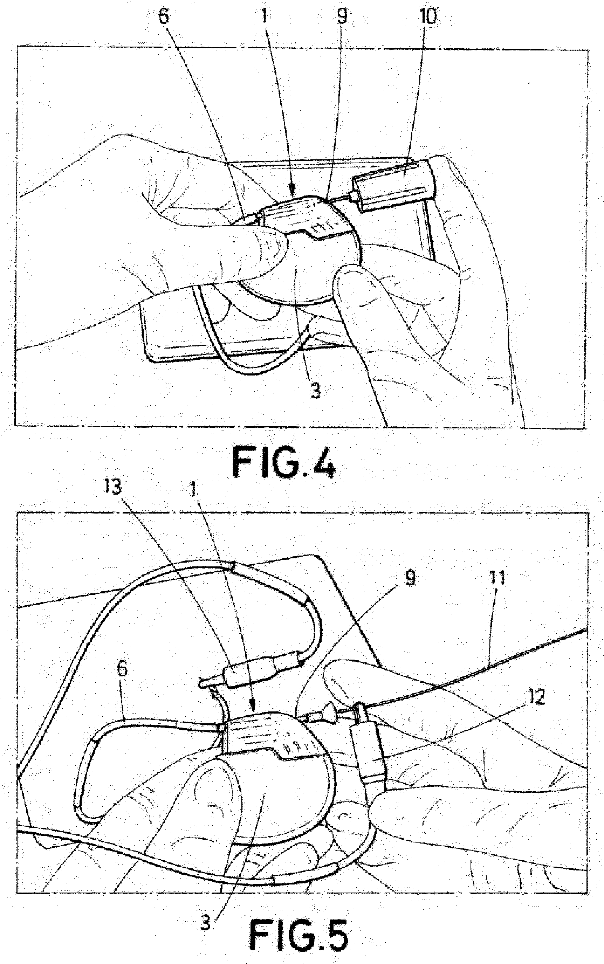

In this sense, as seen in Figures 1 to 5 the connector block (1) at hand is formed by a body (2) made of polyurethane or silicone which is coupled to the casing of the impulse generator (3) of an implantable electrical stimulation device (4) of the pacemaker-type, defibrillator-type, resynchronizer-type, neurostimulator-type, or of another type, housing therein one or more connections (5) for the electrodes (6) for connection thereof to the battery and circuit of said generator (3), and the set screws (7) of said connections (5) housed in a valve (8) preventing the passage of fluids to the inside thereof.

Based on said already known configuration, said connector block (1) is characterized by furthermore having at least one auxiliary port (9) which, provided with an auxiliary valve (8'), provides access to the mentioned connections (5) for the electrodes (6).

More specifically, said auxiliary port (9) is formed as an opening which is located in the rear portion of the connection (5) for one or more electrodes (6), i.e., in the part opposite the inlet/outlet of the electrodes (6), there preferably being an auxiliary port (9) for each connection (5) for the electrode (6) incorporated in the body (2) of the connector block (1). Therefore, although the example depicted in Figures 2 and 3, respectively, shows a known example of a connector block (1') and an example of the connector block (1) according to the invention, both with a single connection (5) to a single electrode (6), there are devices in which the connector block has more than one electrode (6) and the corresponding connection (5).

In any case, said opening is arranged and formed such that it allows inserting both a dynamometric key (10) to thrust the distal end and release the electrode (6), and a stylet (11) which, when going through said opening, will be in electrical contact with the inner lead, and replacing the impulse generator (3), maintaining electrical stimulation, by means of connection to measurement cables, the cathode (12) being connected to the stylet (11) and the anode (13) being connected to the patient's skin, as described in preceding sections.

It must also be highlighted that the auxiliary valve (8') of the auxiliary port (9) preferably has similar dimensions and characteristics as the valve (8) of the set screws (7) incorporated in the body (2) of the connector (1) to prevent fluid from entering the connection (5).

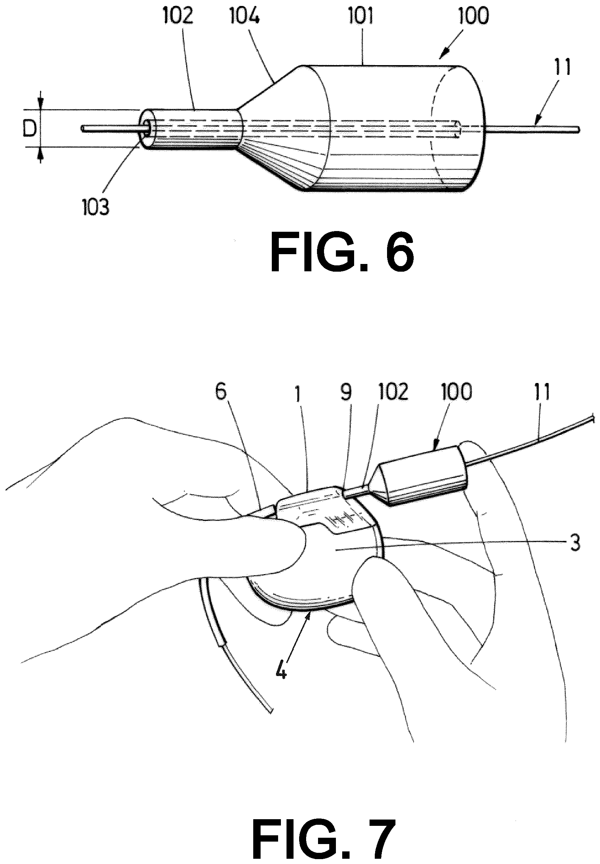

In turn, Figures 6 and 7 show how the tool (100) for procedures for replacing devices (4) having the connector block (1), which is also the object of the present invention, comprises a main body (101) made of plastic or another insulating material, formed by way of a gripping part, having a front portion (102) with a hollow, narrow, and elongated cylinder shape, also made of the same material, acting as a thruster, consisting preferably of a cylinder the diameter or outer width (D) of which is suitable for allowing the insertion thereof in the auxiliary port (9) of the connector block (1) of the implantable electrical stimulation device (4) to enable applying thrust on the distal end of the electrode (6), there being a longitudinal conduit (103) going through both the main body (101) and the front thrusting portion (102), said conduit being suitable for the insertion of a stylet (11) therethrough such that it is fitted therein but with freedom of movement.

In the preferred embodiment, the main body (101) acting as a gripping part is cylindrical but has a larger diameter than the front portion (102), there being a frustoconical attachment area (104) between both.

Figure 7 shows the way of using the tool (100) in the device (4) which, as indicated above, comprises a connector block (1) which is coupled to the casing of the impulse generator (3), housing therein one or more connections (5) for the electrodes (6) for connection thereof to the battery and circuit of said generator (3), where at least one auxiliary port (9) providing access to the mentioned connections for the electrodes (6) has been envisaged.

The end of the stylet (11) previously threaded in the tool (100) is inserted through the auxiliary port (9). Since the stylet (11) is in contact with the inner lead of the electrode to be replaced, a path for bringing about monopolar stimulation by means of using an analyzer is attained.

By sliding the tool (100) on the stylet (11) and inserting the front portion (102) or thruster through the same auxiliary port (9), the electrode is thrust mechanically in the case of adherence or a certain degree of locking.

Having sufficiently described the nature of the present invention as well as the way of putting it into practice, it is not considered necessary to further describe the invention so that a person skilled in the art can comprehend the scope thereof and the advantages derived from it, stating that within its essential nature, the present invention could be carried out to practice in other embodiments differing in detail from that indicated by way of example, and such embodiments would also be granted the protection that is sought provided that the fundamental principle thereof is not altered, changed or modified.

## Claims

1. Connector block for implantable electrical stimulation devices being formed by a body (2) which is coupled to the impulse generator (3) of an implantable electrical stimulation device (4) of the pacemaker-type, defibrillator-type, resynchronizer-type, neurostimulator-type, or of another type, housing one or more connections (5) for electrodes (6) and set screws (7) protected in a valve (8) preventing the passage of fluids to the inside thereof, **characterized by** furthermore having at least one auxiliary port (9) provided with an auxiliary valve (8') for inserting both a dynamometric key (10) to thrust the connection (5) and release the electrode (6), and a stylet (11) which will be in electrical contact with the inner lead, and for replacing the impulse generator (3), maintaining electrical stimulation, by means of connection to measurement cables, the cathode (12) being connected to the stylet (11) and the anode (13) being connected to the patient's skin.

2. Connector block for implantable electrical stimulation devices according to claim 1, **characterized in that** said auxiliary port (9) consists of an opening which is located in the rear portion of the connection (5) for one or more electrodes (6), i.e., in the portion opposite the outlet of the electrodes (6).

3. Connector block for implantable electrical stimulation devices according to claim 1 or 2, **characterized in that** it has an auxiliary port (9) for each connection (5) for the electrode (6) incorporated in the body (2) of the connector block (1).

4. Connector block for implantable electrical stimulation devices according to any of claims 1 to 3, **characterized in that** the auxiliary valve (8') of the auxiliary port (9) has similar dimensions and characteristics as the valve (8) of the set screws (7) incorporated in the body (2) of the connector (1) to prevent fluid from entering the connection (5).

5. Specific tool for use in procedures for replacing devices having a connector block like the one described in any of claims 1 to 4, **characterized by** comprising a main body (101) made of an insulating material, formed by way of a gripping part having a front thrusting portion (102) made of the same material, the outer width (d) of which is suitable for allowing the insertion thereof in the auxiliary port (9) of the device (4) and applying thrust on the distal end of an electrode (6), a longitudinal conduit (103) going through both the main body (101) and the front thrusting portion (102) and being suitable for the insertion therethrough of a semi-rigid stylet (11) such that it is fitted therein but with freedom of movement.

6. Specific tool according to claim 5, **characterized in that** the main body (101) and the front thrusting portion (102) are made of plastic.

7. Specific tool according to claim 5 or 6, **characterized in that** the front thrusting portion (102) is cylindrical.

8. Specific tool according to claim 7, **characterized in that** the main body (101) acting as a gripping part is cylindrical, with a diameter greater than the front portion (102), there being a frustoconical attachment area (104) between both.
